# EUROPEAN PATENT APPLICATION

(11) **EP 4 732 791 A1**
(43) Date of publication of application: **29.04.2026**
(21) Application number: 24844616.3
(22) Date of filing: 10.07.2024
(51) Int. Cl.: A61B 17/221

(54) **THROMBUS REMOVAL SYSTEM AND ASSISTED RECOVERY SYSTEM**

(30) Priority: 26.07.2023 CN 202310928056
(71) Applicant: Shanghai MicroPort Medical (Group) Co., Ltd., Shanghai 201203 (CN)
(72) Inventor: GENG, Kangkang, Shanghai 201203 (CN); ZHOU, Qi, Shanghai 201203 (CN); WU, Jintian, Shanghai 201203 (CN); HUANG, Haiyong, Shanghai 201203 (CN)
(74) Representative: Marks & Clerk LLP
(86) International application number: PCT/CN2024/104636
(87) International publication number: WO 2025/020922

(57) **Abstract**

The present invention relates to a thrombus removal system and an assisted retrieval system. The thrombus removal system includes a thrombectomy device including a push-pull guide wire and a thrombectomy stent, which are sequentially coupled along an axis of the thrombectomy device. The assisted retrieval system includes a retrieval catheter, a push-pull mechanism and a self-expanding stent. A proximal end of the self-expanding stent is coupled to a distal end of the push-pull mechanism. The push-pull mechanism is configured to be slidably disposed within the retrieval catheter and to push or pull the self-expanding stent along an axis of the retrieval catheter. The self-expanding stent comprises a collapsed configuration for its delivery in the retrieval catheter and an expanded configuration when it is partially released from the retrieval catheter. The assisted retrieval system is configured to be moved along the push-pull guide wire and to be located on a proximal side of the thrombectomy stent, thereby enabling the self-expanding stent to be partially released from the retrieval catheter to expand. After the thrombectomy stent captures a target object, it is proximally retracted by the push-pull guide wire at least partially into the self-expanding stent. Thus, the retraction of the thrombectomy stent occurs under the protection of the self-expanding stent, which prevents any thrombus from entering a branch or distal blood vessel.

## Description

### TECHNICAL FIELD

The present invention relates to the field of medical devices and, in particular, to a thrombus removal system and an assisted retrieval system.

### BACKGROUND

An embolism is a blockage of a blood vessel by foreign material that has been carried in the circulating blood without being dissolved therein. When a coronary thrombosis occurs, the patient may experience symptoms such as sudden chest pain or discomfort, shortness of breath, sweating, fainting, nausea or vomiting, which can ultimately lead to myocardial ischemia and myocardial infarction. When a thrombus in the cerebrovascular system blocks blood flow in the brain, the patient may experience symptoms such as a sudden severe headache, dizziness, inability to speak, limb weakness or numbness, blurred, lost or double vision, facial numbness or drooping, impaired consciousness or coma, which can ultimately lead to ischemic necrosis of local brain tissue.

Although most thrombi in intracranial and coronary arteries are hard thrombi, but there are also some soft and semi-hard or semi-soft ones. Fresh thrombi are soft and easy to lyse. They are the so-called red thrombi and are general softness and fragility. Due to increasing polymerization of platelets and fibrin, such thrombi will become harder and harder over time and finally turn into the white thrombi that are rich in fibrin, highly viscoelastic and compression-resistant. Stent thrombectomy is a common modality of mechanical thrombectomy, which can be used to quickly recanalize an occluded blood vessel with high success. Most existing stents for this purpose are self-expanding ones. Such a stent is crimped into a microcatheter before use, which is advanced through a thrombus and then retracted, during use. When not constrained any more, the thrombectomy stent expands by itself. In this process, struts of the stent gradually cut into and embed in the thrombus, or the thrombus is broken up, which enter the interior of the thrombectomy stent. After that, a guide wire is proximally pulled, retracting the thrombectomy stent together with the thrombus back into the catheter.

However, such conventional thrombectomy stents remain unsatisfactory in performance in removing thrombi, in particular those in intracranial and coronary arteries, because these blood vessels are tortuous and thrombi therein may have become plaques or calcified, adding difficulties to thrombectomy. In addition, a thrombectomy stent may be pulled a long distance in order to be retracted from an occluded site back into a retrieval catheter. In this process, a red, soft thrombus is prone to disruption into smaller particles, which tend to escape into distal vessels, forming new emboli. In particular, intracranial arteries have many branches, and coronary arteries have even more branches. During the retraction of a thrombectomy stent, a thrombus may fall off the stent and enter such a branch vessel, or a distal vessel, possibly eventually developing into a new embolus. When this occurs to a coronary artery, a threat may arise to the patient's life.

It should be noted that the information disclosed in this Background section is merely intended to provide a better understanding of the general context of the present application and should not be taken as an acknowledgement or any form of admission that the information forms part of the common general knowledge of those skilled in the art.

### SUMMARY OF THE INVENTION

In view of this, it is an object of the present invention to provide a thrombus removal system and an assisted retrieval system, which can effectively prevent the entry into a branch or distal vessel of thrombus that has fallen off a thrombectomy stent during retraction of the stent.

To this end, the present invention provides a thrombus removal system comprising:
a thrombectomy device comprising a push-pull guide wire and a thrombectomy stent, which are sequentially coupled along an axis of the thrombectomy device; and
an assisted retrieval system comprising a retrieval catheter, a push-pull mechanism and a self-expanding stent, a proximal end of the self-expanding stent coupled to a distal end of the push-pull mechanism, the push-pull mechanism configured to be slidably disposed within the retrieval catheter and to push or pull the self-expanding stent along an axis of the retrieval catheter, the self-expanding stent switchable comprising a collapsed configuration during delivery thereof in the retrieval catheter and an expanded configuration when being partially released from the retrieval catheter,
the assisted retrieval system configured, after the thrombectomy stent captures a target object, to be moved along the push-pull guide wire and to be located on a proximal side of the thrombectomy stent, thereby enabling the self-expanding stent to be partially released from the retrieval catheter to expand,
the thrombectomy stent configured, after capturing the target object, to be pulled by the push-pull guide wire proximally toward the assisted retrieval system, and to be at least partially retracted into the self-expanding stent through a distal opening of the self-expanding stent that has been expanded for retrieval.

In one embodiment, after the thrombectomy stent is at least partially retracted into the self-expanding stent, the thrombectomy device and the assisted retrieval system may be configured to be proximally moved together into an outer sheath, or wherein the thrombectomy stent is proximally moved and passed through the self-expanding stent into the retrieval catheter.

In one embodiment, the self-expanding stent may consist of a braided mesh and comprises a slit extending through the self-expanding stent along an axis thereof.

In one embodiment, the braided mesh may be switchable between a rolled configuration and an unrolled configuration, making the self-expanding stent able to adapt its geometry to the size of a tubular lumen where it is located.

In one embodiment, the self-expanding stent may comprise a crimped section, a tapered section and a cylindrical support section, which are sequentially joined along the axis of the self-expanding stent, the crimped section disposed in the retrieval catheter and coupled to the push-pull mechanism, both the tapered and support sections can be released from the retrieval catheter to expand.

In one embodiment, the tapered section may be provided with a plurality of radiopaque markers arranged circumferentially around its proximal edge.

In one embodiment, the push-pull mechanism may comprise a push rod coupled at a distal end thereof to a proximal end of the self-expanding stent. Alternatively, the push-pull mechanism may comprise a push rod and a pull wire, a distal end of the pull wire coupled to the proximal end of the self-expanding stent, wherein a proximal end of the pull wire passes through the push rod and extends to a proximal end of the retrieval catheter.

In one embodiment, the self-expanding stent may be provided with a hydrophilic coating layer on at least part of its exterior surface. Alternatively or additionally, the retrieval catheter may be provided with a hydrophilic coating layer on inner wall of a distal end thereof.

In one embodiment, the thrombectomy stent may comprise a first thrombectomy stent, a proximal end of the first thrombectomy stent coupled to a distal end of the push-pull guide wire, the first thrombectomy stent comprising a tubular structure consisting of a plurality of interconnected closed mesh openings including first mesh openings, second mesh openings and third mesh openings, the first and third mesh openings having a smaller area than the second mesh openings, the first mesh openings having no overlap with respect to the second or third mesh openings, the third mesh openings is arranged within the second mesh openings, at least two second mesh openings spanning at least the circumference of an exterior surface of the first thrombectomy stent.

In one embodiment, the second mesh openings may have an area which is 2.5 to 6 times an area of the first mesh openings.

In one embodiment, a position of the first thrombectomy stent corresponding to the second mesh opening may be provided with radiopaque feature element.

In one embodiment, the first thrombectomy stent may comprise a proximal portion, a middle portion and a distal portion, which are joined sequentially, wherein the first mesh openings are distributed on the proximal portion and the distal portion, wherein the second mesh openings are distributed on the middle portion, and wherein the first mesh openings in the distal portion are smaller in area than the first mesh openings in the proximal portion.

In one embodiment, a plurality of second mesh openings may be distributed on different circumferences of the first thrombectomy stent, wherein the second mesh openings distributed on different circumferences have overlap portions along an axis and/or circumference of the first thrombectomy stent.

In one embodiment, the thrombectomy stent may further comprise a second thrombectomy stent formed by a braided dense mesh, a proximal end of the second thrombectomy stent flexibly coupled to a distal end of the first thrombectomy stent, the second thrombectomy stent able to occlude the self-expanding stent on a distal side of the first thrombectomy stent after the first thrombectomy stent being retracted into the self-expanding stent.

In one embodiment, the second thrombectomy stent may be configured to occlude a distal opening of the self-expanding stent at a distal side of the self-expanding stent, or wherein the second thrombectomy stent is at least partially retracted into the self-expanding stent for occlusion.

The present invention also provides an assisted retrieval system of the same inventive concept, which comprises a retrieval catheter, a push-pull mechanism and a self-expanding stent. A proximal end of the self-expanding stent is coupled to a distal end of the push-pull mechanism. The push-pull mechanism is configured to be slidably disposed within the retrieval catheter and to push or pull the self-expanding stent along an axis of the retrieval catheter. The self-expanding stent comprises a collapsed configuration during delivery thereof in the retrieval catheter and an expanded configuration when being partially released from the retrieval catheter.

The assisted retrieval system is configured to, after a thrombectomy stent captures a target object, be moved along a push-pull guide wire and be located on a proximal side of the thrombectomy stent, thereby enabling the self-expanding stent to be partially released from the retrieval catheter to expand and enabling at least a part of the self-expanding stent to be retracted into the self-expanding stent through a distal opening of the self-expanding stent that has been expanded for retrieval.

The present invention further provides a method for removing a thrombus using the thrombus removal system as defined above. The method comprises:
delivering the thrombectomy stent to a site in need of thrombectomy, using an interventional catheter;
withdrawing the interventional catheter and causing the thrombectomy stent to self-expand and capture the thrombus;
after the thrombus is captured, moving the retrieval catheter that contains the self-expanding stent along the push-pull guide wire, locating it on a proximal side of the thrombectomy stent and pushing the self-expanding stent toward a distal end of the retrieval catheter through the push-pull mechanism, thereby enabling the self-expanding stent to be partially released from the retrieval catheter to expand;
subsequently, proximally pulling the thrombectomy stent through the push-pull guide wire to until it is at least partially retracted into the self-expanding stent; and
then retracting the thrombectomy device and the assisted retrieval system optionally together into an outer sheath.

As noted above, the present invention provides a thrombus removal system including a thrombectomy device and an assisted retrieval system. During retraction of the thrombectomy device together with a thrombus captured therein, it can be at least partially received in an expanded portion of a self-expanding stent in the assisted retrieval system. With this arrangement, the thrombectomy stent can be retracted from an occluded site into the retrieval catheter while being protected by the self-expanding stent. As such, a thrombus that falls off the thrombectomy stent during the retraction can be effectively prevented from entering a branch or distal blood vessel, resulting in higher thrombectomy efficiency and success, a reduced risk of secondary stroke and increased surgical safety.

Since the assisted retrieval system is of the same inventive concept as the thrombus removal system, it has all the advantages of the latter. For brevity, these advantages are not repeated here.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 shows a schematic representation of a thrombectomy device according to an embodiment disclosed herein;
Fig. 2 shows a schematic representation of another thrombectomy device according to an embodiment disclosed herein;
Fig. 3 is a schematic flattened view of a first thrombectomy stent according to an embodiment disclosed herein;
Fig. 4 is a schematic perspective view of the first thrombectomy stent;
Fig. 5 is a schematic elevation view of the first thrombectomy stent;
Fig. 6 is a schematic flattened view of another first thrombectomy stent according to an embodiment disclosed herein;
Fig. 7 is a schematic perspective view of the other first thrombectomy stent;
Fig. 8 is a schematic side view of a first thrombectomy stent according to an embodiment disclosed herein;
Fig. 9 is a schematic plan view of an assisted retrieval system in an initial configuration according to an embodiment disclosed herein;
Fig. 10 is a schematic plan view of the assisted retrieval system during operation;
Fig. 11 is a schematic enlarged partial view of a tapered section of a self-expanding stent according to an embodiment disclosed herein, showing a radiopaque marker provided on a proximal edge of the tapered section;
Fig. 12 is a schematic flattened view of the self-expanding stent;
Fig. 13 shows a cross-section of the self-expanding stent of Fig. 12, taken along A-A;
Fig. 14 shows a cross-section of the self-expanding stent of Fig. 12, taken along B-B;
Fig. 15 is a schematic perspective view of a released expandable segment of the self-expanding stent according to an embodiment disclosed herein;
Fig. 16 shows a schematic representation of a self-expanding stent according to an embodiment disclosed herein;
Fig. 17 is a schematic side view of a self-expanding stent according to an embodiment disclosed herein;
Figs. 18a to 18d schematically illustrate how a self-expanding stent is rolled and overlapped, in accordance with an embodiment disclosed herein;
Figs. 19 to 22 schematically illustrate steps in a process for removing a thrombus from a patient's blood vessel using a thrombus removal system according to an embodiment disclosed herein; and
Fig. 23 shows a schematic representation of yet another thrombectomy device according to an embodiment disclosed herein, which includes only a first thrombectomy stent.

### List of Reference Numerals

10-thrombectomy device; 11-push-pull guide wire; 12-coupling member; 13-thrombectomy stent; 13A-strut; 130A-first strut; 130B-second strut; 130C-joint of first and second struts; 130D-joint of struts in second mesh openings; 13B-straight strut; 13C-tapered closed end; 131-first thrombectomy stent; 131a-first mesh opening; 131b-second mesh opening; 131c-third mesh opening; 132-second thrombectomy stent; 14-radiopaque ring; 15-structural member; 16-opening inlet; 17-overlap portion; 18-free projection; 30-assisted retrieval system; 31-retrieval catheter; 32-push-pull mechanism; 321-push rod; 322-pull wire; 33-self-expanding stent; 33a-distal opening; 33b-slit; 331-crimped section; 332-tapered section; 333-support section; 334-radiopaque marker; 100-microcatheter; 101-thrombus; 102-blood vessel wall; 103-branch blood vessel.

### DETAILED DESCRIPTION

Embodiments of the present invention will be described below by way of specific examples. Based on the disclosure and teachings provided herein, a person of ordinary skill in the art will readily realize other advantages and benefits provided by the present invention. The invention may also be otherwise embodied or applied through different embodiments, and various modifications or changes may be made to the details disclosed herein from different points of view, or for different applications, without departing from the spirit of the invention. It should be noted that the accompanying drawings are provided herein merely to schematically illustrate the basic concept of the present invention. Accordingly, they only show components relating to this invention but not necessarily depict all the components as well as their real shapes and dimensions in practical implementations. In practice, the configurations, counts and relative scales of the components may vary arbitrarily and their arrangements may be more complicated.

Objects, advantages and features of the present invention will become more apparent upon reading the following more detailed description of specific embodiments thereof in conjunction with the accompanying drawings. Note that the figures are provided in a very simplified form not necessarily drawn to exact scale for the only purpose of helping explain embodiments of the invention disclosed herein in a more convenient and clearer way. Throughout these drawings, same numerals indicate same elements. Additionally, the use of the terms "first", "second" or the like is intended for illustration only and is not to be construed as denoting or implying relative importance, or as implicitly indicating the number of referenced items. Accordingly, defining an item with "first", "second" or the like is an explicit or implicit indication of the presence of one or at least two such items. As used herein, the term "plurality" is generally employed in the sense of "two or more", unless otherwise specified.

It is noted that, when used herein to describe a medical device intended to be implanted into the body of a human or animal, the term "proximal end" or "proximally" generally refers to the end of the device that is closer to an operator during the implantation, and "distal end" or "distally" generally refers to its end farther away from an operator during the implantation. Additionally, when used herein to describe a medical device being delivered, the term "axial direction" generally refers to a lengthwise direction of the device, "radial direction" to a direction perpendicular to the "axial direction", and "circumferential" to a direction about its central axis.

It is a principal object of the present invention to provide a thrombus removal system and an assisted retrieval system, which overcomes at least the prior art problem that thrombus that has fallen off a thrombectomy stent during retraction thereof tends to enter a branch or distal vessel.

The invention is described below with reference to the accompanying drawings. Although the following description is set forth in the context of a thrombectomy device being delivered using a microcatheter, those skilled in the art will recognize that the delivery of the thrombectomy device may indeed also be accomplished using another interventional catheter than a microcatheter, such as a guide catheter. Those skilled in the art will know how to adapt the description below to the case of such another interventional catheter than a microcatheter.

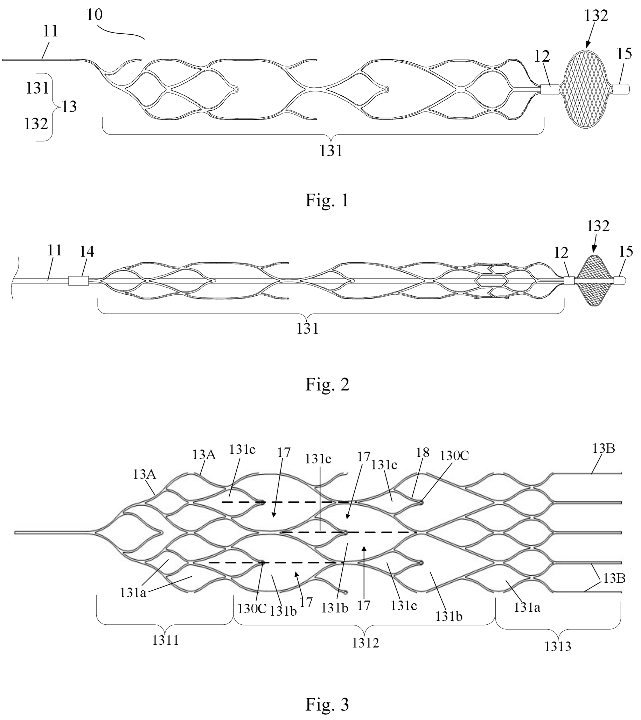

Referring to Figs. 1 to 23, embodiments disclosed herein provide a thrombus removal system including a thrombectomy device 10 and an assisted retrieval system 30. The thrombectomy device 10 includes a push-pull guide wire 11 and a thrombectomy stent 13, which are sequentially coupled to each other along an axis of the thrombectomy device 10. The push-pull guide wire 11 is configured with a degree of flexibility and a degree of bendability and can be used to push the thrombectomy device 10 to release it from a microcatheter 100. It can also be used to pull the thrombectomy device 10 to retract it back into the assisted retrieval system 30. In practice, the assisted retrieval system 30 may be able to move along the push-pull guide wire 11 towards or away from the thrombectomy stent 13.

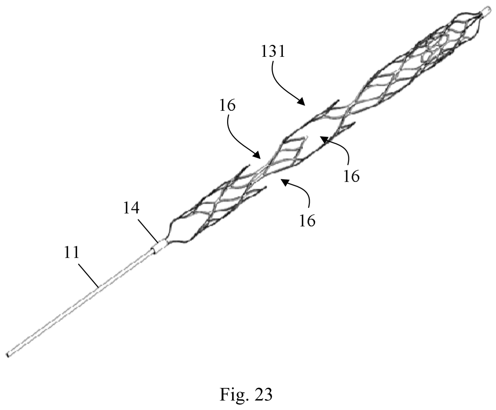

Referring to Figs. 1 and 2, in one embodiment, the thrombectomy stent 13 includes a first thrombectomy stent 131 and a second thrombectomy stent 132. As shown in Fig. 23, in an alternative embodiment, the thrombectomy stent 13 includes only a first thrombectomy stent 131. The invention is exemplified in the context of the thrombectomy device 10 including a first thrombectomy stent 131 and a second thrombectomy stent 132.

As shown in Figs. 1 and 2, the thrombectomy stent 13 may include a first thrombectomy stent 131 and a second thrombectomy stent 132. The first thrombectomy stent 131 is a tubular structure consisting of multiple interconnected closed mesh openings (i.e., the first thrombectomy stent 131 is a tubular structure with mesh openings), and the second thrombectomy stent 132 consists of a braided mesh. A proximal end of the first thrombectomy stent 131 is coupled to a distal end of the push-pull guide wire 11, and a distal end of the first thrombectomy stent 131 is coupled to a proximal end of the second thrombectomy stent 132. Preferably, the distal end of the first thrombectomy stent 131 is flexibly coupled to the proximal end of the second thrombectomy stent 132. Advantages of such a flexible coupling include allowing the distal end of the first thrombectomy stent 131 and the proximal end of the second thrombectomy stent 132 to deflect with a great bending angle, to adapt themselves to large curves of a blood vessel segment, thereby reducing damage of a wall of the blood vessel during the delivery of the device in the tortuous blood vessel. It will be understood that flexibly coupling the first and second thrombectomy stents 131, 132 to each other can mitigate mutual interference of them, reducing damage caused by the thrombectomy device 10 to a tortuous blood vessel during its advancement therein. In addition, the second thrombectomy stent 132 can effectively capture an escaping thrombus or thrombus fragments, preventing the escape of the thrombus to branch 103 and/or distal blood vessel.

In order to remove a thrombus, the thrombectomy device 10 may be delivered to the site in need of thrombectomy using a microcatheter 100. To this end, the thrombectomy device 10 may comprise a collapsed configuration in which it is crimped and received within the microcatheter 100, and a fully expanded configuration after being pushed out of the microcatheter 100 from a distal end thereof. When the thrombectomy device 10 is crimped, an overall size thereof is reduced, thereby allowing it to be placed in the microcatheter 100. The microcatheter 100 is adapted to deliver the thrombectomy device 10 in the collapsed configuration to a site in need of thrombectomy. Once released from the microcatheter 100, the thrombectomy device 10 will be no longer constrained by the microcatheter 100 and expand by itself.

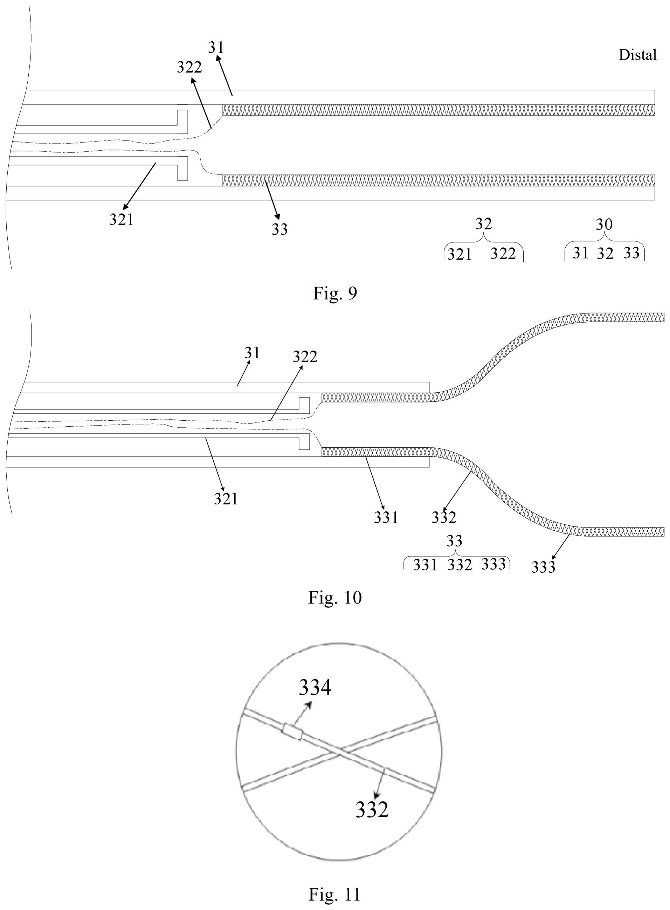

Referring to Figs. 9 to 10, the assisted retrieval system 30 includes a retrieval catheter 31, a push-pull mechanism 32 and a self-expanding stent 33. A proximal end of the self-expanding stent 33 is coupled to a distal end of the push-pull mechanism 32. The retrieval catheter 31 comprises a lumen extending therethrough along its own axis. The push-pull mechanism 32 is configured to be slidably disposed within the lumen of the retrieval catheter 31. The push-pull mechanism 32 is able to push or pull the self-expanding stent 33 along the axis of the retrieval catheter 31, thereby causing the self-expanding stent 33 partially out of the retrieval catheter 31, or retracting an already expanded portion of the self-expanding stent 33 back into the retrieval catheter 31.

To this end, the self-expanding stent 33 may comprise a collapsed configuration (Fig. 9) for its delivery within the retrieval catheter 31 and an expanded configuration (Fig. 10) in which it is partially released from the retrieval catheter 31. With this arrangement, after the thrombectomy device 10 captures a thrombus and is to be retrieved, the thrombectomy stent 13 can be retracted at least partially back into the portion of the self-expanding stent 33 that has been released from the retrieval catheter 31 and expanded. To this end, the self-expanding stent 33 may comprise a lumen allowing passage of the thrombectomy stent 13 therethrough. After successfully capturing a thrombus, the thrombectomy stent 13 can be retracted back into the lumen of the self-expanding stent 33, or through the lumen into the retrieval catheter 31.

In practical use, the entire first thrombectomy stent 131 may be retracted into the self-expanding stent 33, with the second thrombectomy stent 132 being not, partially or entirely received in the self-expanding stent 33 while occluding a distal opening 33a of the self-expanding stent 33. Alternatively, both the first and second thrombectomy stents 131, 132 may be retracted through the self-expanding stent 33 into the retrieval catheter 31. With this arrangement, during thrombus capture and retraction of the first thrombectomy stent 131, the second thrombectomy stent 132 is able to block thrombus distally. Moreover, after the first thrombectomy stent 131 is retracted into the self-expanding stent 33, the second thrombectomy stent 132 occludes the self-expanding stent 33 on the distal side of the first thrombectomy stent 131, further blocking escape of any thrombus therefrom. Thus, the second thrombectomy stent 132 is allowed to occlude the distal opening 33a of the self-expanding stent 33 on the distal side thereof while not, partially or entirely received in the self-expanding stent 33.

Whether there is one or more thrombectomy stents 13, the assisted retrieval system 30 is configured to be moved along the push-pull guide wire 11 after the microcatheter 100 for delivering the thrombectomy stent(s) 13 is withdrawn and the thrombectomy stent(s) 13 captures the thrombus, and to be positioned on a proximal side of the thrombectomy stent(s) 13. After that, it is manipulated to control a portion of the self-expanding stent 33 out of the retrieval catheter 31, followed by expansion of the released portion. The thrombectomy stent(s) 13 that captured the thrombus is/are then pulled by the push-pull guide wire 11 proximally toward the assisted retrieval system 30 and it is at least partially retracted into the expanded self-expanding stent 33 through the distal opening 33a thereof for retrieval.

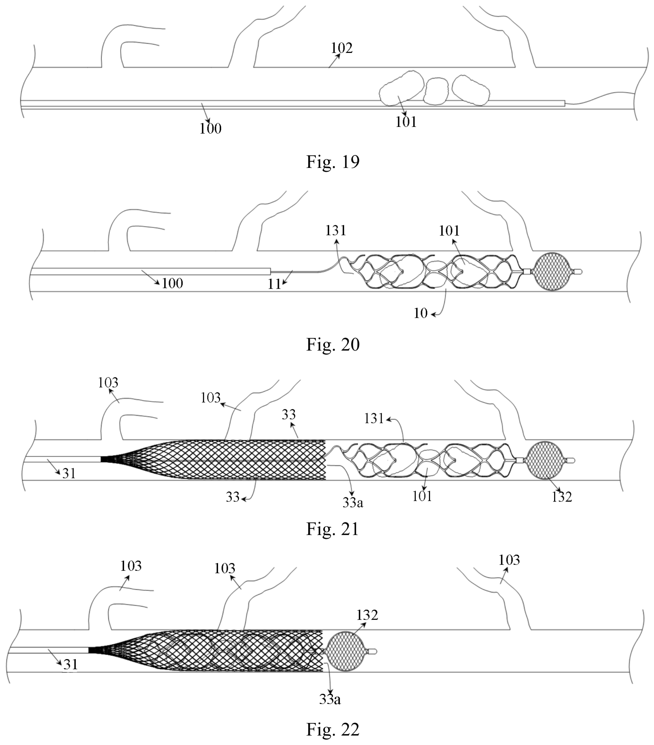

A thrombus removal method and steps according to embodiments disclosed here are further described below with reference to Figs. 19 to 22.

As shown in Fig. 19, the microcatheter 100 containing the collapsed thrombectomy device 10 is delivered to a distal end of an outer sheath (not shown) and then further advanced distally to cause the microcatheter 100 to extend out of a distal end of the outer sheath and to pass through a gap left between a thrombus 101 and a blood vessel wall 102. At this point, the thrombus 101 is substantially located at a position where the first thrombectomy stent 131 is located.

Once the first thrombectomy stent 131 is roughly located at the thrombus 101 by the microcatheter 100, as shown in Fig. 20, with the push-pull guide wire 11 being held stationary, the microcatheter 100 is retracted proximally until it is withdrawn from the patient's body. In the process of retracting the microcatheter 100, the thrombectomy device 10 gradually self-expands and comes into contact with the thrombus 101. As a result, the thrombus 101 is captured in the first thrombectomy stent 131.

Once the thrombus is captured, as shown in Fig. 21, the retrieval catheter 31 containing the self-expanding stent 33 is advanced along the push-pull guide wire 11 to approach the first thrombectomy stent 131 as much as possible. After that, it is held stationary, and the push-pull mechanism 32 is then manipulated to push the self-expanding stent 33 toward a distal end of the retrieval catheter 31. Once the self-expanding stent 33 is partially released and expands into contact with the blood vessel wall 102, the thrombectomy device 10 is immediately pulled proximally by manipulating the push-pull guide wire 11. As a result, the thrombectomy device 10 is caused to proximally move in the direction opposite to blood flow. In this process, fragments of the thrombus may fall off the first thrombectomy stent 131 and flows in the direction of blood flow toward the second thrombectomy stent 132. However, since the second thrombectomy stent 132 consists of a braided mesh and has expanded into contact with the blood vessel wall 102, the escaping fragments of the thrombus can be effectively captured by the second thrombectomy stent 132.

As shown in Fig. 22, the thrombectomy device 10 may be retracted so that the entire first thrombectomy stent 131 is moved into the lumen of the expanded portion of the self-expanding stent 33, with the second thrombectomy stent 132 optionally remaining outside of the self-expanding stent 33 while closing its distal opening 33a. Subsequently, the self-expanding stent 33, the thrombectomy device 10 and the retrieval catheter 31 may be together retracted proximally until they are entirely received in the outer sheath.

However, in alternative implementations, the thrombectomy stent 13 may be proximally moved through the self-expanding stent 33 directly into the retrieval catheter 31. For example, the thrombectomy device 10 with captured thrombus may be entirely retracted through the lumen of the self-expanding stent 33 directly into the retrieval catheter 31. In these cases, after both the first and second thrombectomy stent 131, 132 are moved into the self-expanding stent 33, the thrombectomy device 10 may be further pulled proximally, until it is retracted into the retrieval catheter 31. After that, the thrombectomy device 10 may be retracted, together with the assisted retrieval system 30, into the outer sheath (or simply the "sheath"), to withdrawal from the patient's body. In these cases, the retrieval catheter 31 is desirably selected as one with a larger diameter, in order to allow the thrombectomy device to be retracted into the retrieval catheter 31. This can also provide protection for the thrombectomy stent 13 and keep the captured thrombus always in the self-expanding stent 33 during the retraction of the thrombectomy device 10, reducing the risk of the thrombus falling off and entering branch 103 and/or distal blood vessels during the retraction, possibly because the retraction occurs in a tortuous blood vessel, or because the lesion is a plaque or calcified.

Thus, the thrombectomy stent 13 as discussed above can be retracted from an occluded site into the retrieval catheter 31 while being protected by the self-expanding stent 33. As such, a thrombus that falls off the thrombectomy stent 13 during the retraction can be effectively prevented from entering a branch 103 or distal blood vessel, resulting in higher thrombectomy efficiency and success, a reduced risk of secondary stroke and increased surgical safety.

The self-expanding stent 33 consists essentially of a braided mesh. However, it may also be fabricated by cutting a tube, without departing from the scope of the present invention. The self-expanding stent 33 may be simply a bare stent (without a graft), or a stent graft. Preferably, the self-expanding stent 33 is a braided stent consisting of a metal or polymer wire with hyperelasticity and good shape memory properties and has been set in shape. The self-expanding stent 33 made of braided mesh provides better protection for the thrombectomy stent 13 during retraction, thereby preventing the escape of a thrombus during retraction of the thrombectomy stent 13.

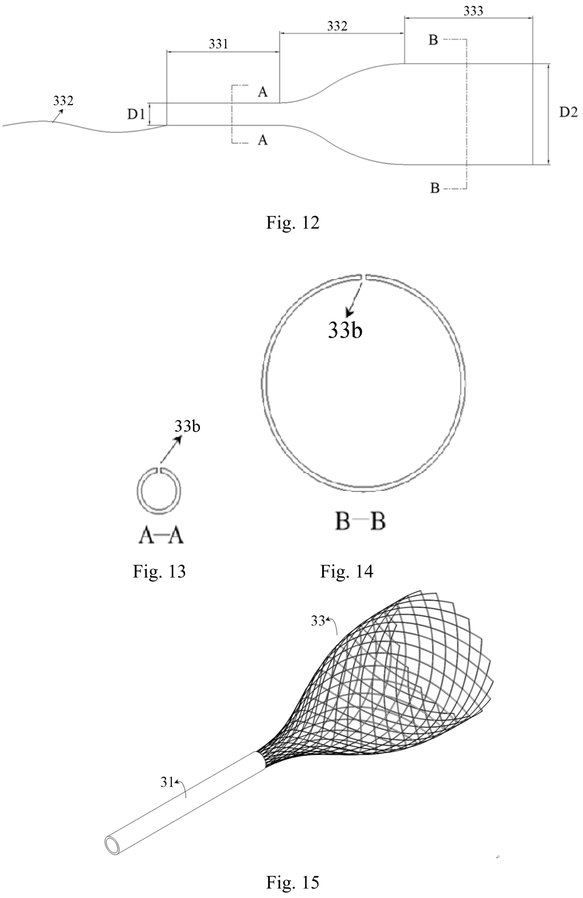

Referring to Figs. 12 to 14 and 16 to 17, the self-expanding stent 33comprises a slit 33b extending therethrough along its own axis. To this end, the self-expanding stent 33 may consist, as a whole, of an open braided mesh, which may be switchable between a rolled configuration and an unrolled configuration, making the self-expanding stent 33 able to adapt its own configuration to the size of a tubular lumen where it is in. With this arrangement, the self-expanding stent 33 is allowed to adapt its geometry to tubular lumens of various different sizes, such as differently sized retrieval catheters 31 and blood vessels. Specifically, when placed into a tubular lumen, the self-expanding stent 33 may be adaptively rolled to have an overlap portion, or unrolled to no longer have any an overlap portion. Thus, the self-expanding stent 33 can vary with a diameter of a tubular lumen to which it is applied.

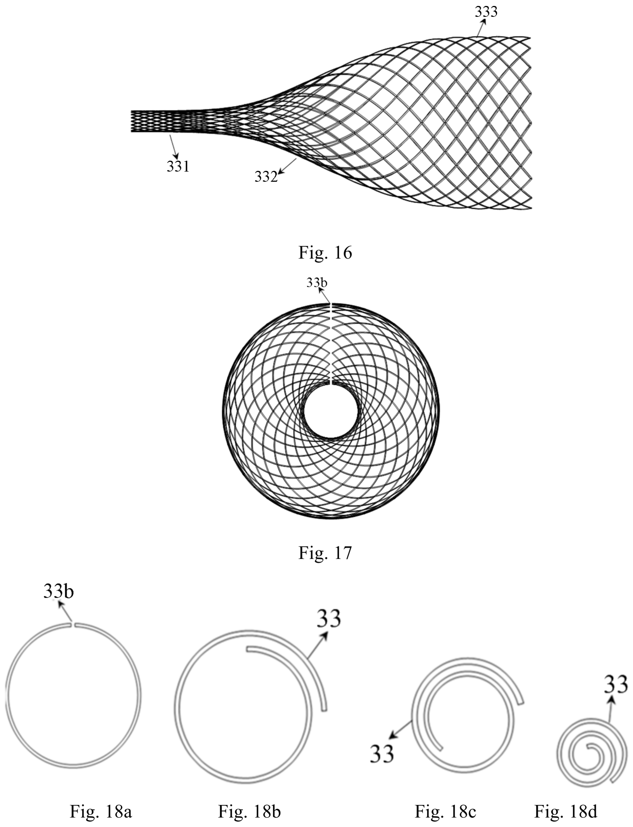

Reference is made to Figs. 18a to 18d for more details of the rolling of the self-expanding stent 33. As can be seen, the self-expanding stent 33 has smaller or no overlap portion, when applied to a tubular lumen with a larger diameter. On the contrary, it is rolled to have larger overlap portion, when applied to a tubular lumen with a smaller diameter. With this arrangement, first, the self-expanding stent 33 can be received in a retrieval catheter 31 with a smaller diameter. Therefore, it can be applied to a blood vessel with a smaller diameter, or advanced to a deeper location in a blood vessel. Second, when used in a blood vessel with a smaller tubular lumen, the self-expanding stent 33 may be rolled to have overlap portion. On the contrary, when used in a blood vessel with a larger tubular lumen, it may comprise an "unrolled" configuration. These make it suitably usable in blood vessels with various different diameters. Third, radial support of the self-expanding stent 33 can be weakened, making it cause less damage to an inner wall of a blood vessel during retraction. However, it will be understood that, in alternative embodiments, the slit 33b may be omitted. In these cases, the self-expanding stent 33 may be in the form of a circumferentially closed cylindrical structure that is radially contractible and expandable.

Referring to Figs. 10 and 12, in a specific embodiment, the self-expanding stent 33 includes a crimped section 331, a tapered section 332 and a cylindrical support section 333, which are sequentially joined along the axis of the self-expanding stent 33. The crimped section 331 is always received in the retrieval catheter 31 and coupled to the push-pull mechanism 32. The tapered section 332 and the support section 333 can be released from the retrieval catheter 31 and then expand. The tapered section 332 and the support section 333 comprise an expandable segment with hyperelasticity and radially self-expanding properties. The support section 333 has an outer diameter slightly greater than a diameter of a blood vessel. In this way, it can closely fit against an inner wall of the blood vessel. Providing the tapered section 332 to the support section 333, the tapered section 332 enables the self-expanding stent 33 to be more smoothly slid out of, or retracted back into the retrieval catheter 31. A smooth transition connection is provided between the tapered section 332 and the support section 333. The outer diameter of the support section 333 is equal to an outer diameter at the joint of the support section 333 and the tapered section 332, which is, in turn, equal to a maximum outer diameter D2 of the tapered section 332. An outer diameter of the tapered section 332 is greater than an outer diameter at the joint of the tapered section 332 and the crimped section 331, which is equal to a minimum outer diameter D1 of the tapered section 332. Restrained by the push-pull mechanism 32, the crimped section 331 always stays within the retrieval catheter 31.

As shown in Fig. 11, in one embodiment, a radiopaque marker 334 is provided on a proximal edge of the tapered section 332 of the self-expanding stent 33. Preferably, a plurality of radiopaque markers 334 is provided in a circumferential arrangement. With this arrangement, an operator (medical staff member) can accurately locate the self-expanding stent 33 during delivery of the self-expanding stent 33 in a surgical procedure, without pushing the self-expanding stent 33 entirely out of the retrieval catheter 31, improving safety and reliability of the procedure. In a specific embodiment, a number of radiopaque markers 334 are provided on braid wire on the proximal edge of the tapered section 332. The radiopaque marker 334 can be secured onto the braided wire via physical crimping. Preferably, the radiopaque markers 334 are equidistantly arranged on a single circumference. The number of radiopaque markers 334 may be in the range of 2 to 8. These radiopaque markers 334 may be made of platinum, gold, iridium, etc., and the present application is not limited to any particular material of them.

Further, the self-expanding stent 33 is provided with a hydrophilic coating layer on at least portion of its exterior surface. The hydrophilic coating layer may cover the entire exterior of the self-expanding stent 33, or only that of the expandable segment. The hydrophilic coating layer on the self-expanding stent 33 may be made of a material including, but not limited to, polyurethane. Other polymeric materials with hydrophilic properties are also possible. Alternatively or additionally, a hydrophilic coating layer may be provided on an inner wall of the retrieval catheter 31 around its distal end, resulting in a reduced coefficient of friction, which allows the expandable segment to be more easily pushed out of the retrieval catheter 31 and can reduce damage caused by the self-expanding stent 33 to a blood vessel in which it is being retracted. The hydrophilic coating layer provided on the inner wall of the retrieval catheter 31 around its distal end may be made of a material including, but not limited to, polyvinylpyrrolidone.

The present application is not limited to any particular structure of the push-pull mechanism 32. Indeed, the push-pull mechanism 32 may be manipulated to push or pull the self-expanding stent 33 by at least one method, as exemplified below.

As shown in Figs. 9 and 10, in an exemplary embodiment, the push-pull mechanism 32 includes a push rod 321 and a pull wire 322. The pull wire 322 is distally coupled to the proximal end of the self-expanding stent 33 and proximally passed through the push rod 321 to a proximal end of the retrieval catheter 31. The proximal end of the pull wire 322 may be coupled to a proximal end of the push rod 321, or not. In the latter case, the proximal end of the pull wire 322 is a free end. In this case, the push rod 321 can be distally brought into abutment with the proximal end of the self-expanding stent 33 and used to distally advance the self-expanding stent 33, and the pull wire 322 can be used to proximally pull the self-expanding stent 33. At least one pull wire 322 may be provided. In the illustrated embodiment, there are multiple pull wires 322. In more detail, several pull wires 322 may be coupled to a proximal end of the crimped section 331 by welding, gluing, riveting, winding or a combination thereof.

In an alternative exemplary embodiment, the push-pull mechanism 32 includes only a push rod 321 having its distal end coupled directly or indirectly to the proximal end of the self-expanding stent 33. In this case, the push rod 321 can be directly manipulated to push or pull the self-expanding stent 33. For ease of connection, the push rod 321 may be coupled to the self-expanding stent 33 with a pull wire 322. That is, the push rod 321 can be fixedly connected to the proximal end of the self-expanding stent 33 by the pull wire 322.

Preferred embodiments of the proposed thrombectomy device 10 will be further described below.

Referring to Figs. 1 and 2, the distal end of the first thrombectomy stent 131 is coupled to the proximal end of the second thrombectomy stent 132 using a coupling member 12. The coupling member 12can be used to couple the distal end of the first thrombectomy stent 131 to the proximal end of the second thrombectomy stent 132 by at least one method. The present application is not limited thereto. The coupling member 12 may be radiopaque or not. Preferably, the coupling member 12 is radiopaque. For example, in one exemplary embodiment, the coupling member 12 is made from a radiopaque metal wire, which is helically wound about an axis thereof on both the distal end of the first thrombectomy stent 131 and the proximal end of the second thrombectomy stent 132. In an alternative exemplary embodiment, the coupling member 12 may consist of a radiopaque ring, which can be crimped onto both the distal end of the first thrombectomy stent 131 and the proximal end of the second thrombectomy stent 132 and thereby coupled them together.

The present application is not limited to any particular approach for coupling the proximal end of the first thrombectomy stent 131 to the distal end of the push-pull guide wire 11. For example, this may be accomplished by winding, crimping, gluing with a medical adhesive, laser welding, fusion welding with a polymeric material, etc. As shown in Fig. 1, in one embodiment, the proximal end of the first thrombectomy stent 131 is welded or glued to the distal end of the push-pull guide wire 11. As shown in Fig. 2, in an alternative embodiment, the proximal end of the first thrombectomy stent 131 is coupled to the distal end of the push-pull guide wire 11 by crimping via a radiopaque ring 14.

As noted above, the first thrombectomy stent 131 is a tubular structure consisting of multiple interconnected closed mesh openings. Each closed mesh opening consists of multiple struts 13A, which are connected end to end, as particularly shown in Fig. 3.

As shown in Figs. 3 to 4 and 7 to 8, in one exemplary embodiment, the distal end of the first thrombectomy stent 131 may comprise multiple straight struts 13B that are circumferentially arranged, the straight struts 13B are converged to form a tapered closed end 13C at the distal end of the first thrombectomy stent 131. The converged structure of the distal end of the first thrombectomy stent 131 allows it to be easily coupled to the proximal end of the second thrombectomy stent 132.

As shown in Figs. 1 and 2, in one exemplary embodiment, the distal end of the second thrombectomy stent 132 is constrained into a tapered shape, with its distal gathered together and secured by a structural member 15 to prevent the braided wires at the distal end from splaying apart. The structural member 15 may be a sleeve, preferably a radiopaque sleeve. The proximal end of the second thrombectomy stent 132 is coaxial with the distal end of the first thrombectomy stent 131.

The first thrombectomy stent 131 may be made of a metal alloy or a polymeric material, which exhibit hyperelasticity and good shape memory properties. Preferably, it is made of a nickel-titanium alloy. Without limitation, the first thrombectomy stent 131 may be formed by cutting a tubular material followed by a thermal shape setting process. The closed mesh openings in the first thrombectomy stent 131 may be of any desirable shape, such as a rhombic, pentagonal or other regular shape, or an irregular shape. The present application is not limited thereto. The first thrombectomy stent 131, when expanded, may have a length and a radial dimension, which depend on a diameter of a target blood vessel and a length of a target thrombus. Optionally, the first thrombectomy stent 131, when fully expanded, may have an axial length ranging from 13 mm to 50 mm and a maximum radial width in the range of 2.0 mm to 6.0 mm. Optionally, the struts 13A may have a width in the range of 0.06 mm to 0.12 mm. The first thrombectomy stent 131 designed with such dimension is able to provide both good support and a desirable radial dimension.

The closed mesh openings in the first thrombectomy stent 131 may be further improved. In particular, as shown in Fig. 3, the closed mesh openings in the first thrombectomy stent 131 may include first mesh openings 131a, second mesh openings 131b and third mesh openings 131c. The first mesh openings 131a and the third mesh openings 131c may have a smaller area than the second mesh openings 131b. The first mesh openings 131a have no overlap with the second mesh openings 131b and the third mesh openings 131c. The third mesh openings 131c are encircled within the second mesh openings 131b. At least two second mesh openings 131b span at least the entire circumference of an exterior surface of the first thrombectomy stent 131. Designing the first thrombectomy stent 131 as a structure consisting of multiple types of mesh openings enables the first thrombectomy stent 131 to effectively remove both soft and hard thrombi with higher thrombectomy efficiency and success. Specifically, since the first mesh openings 131a are smaller in area, they can be more easily embedded into a soft thrombus. The second mesh openings 131b comprise large-area opening inlets 16, which allow the hard and soft thrombi to embed into the second mesh openings 131b or to be trapped into the interior of the first thrombectomy stent 131. The third mesh openings 131c form a spatially warp structure in the second mesh openings 131b to mitigate thrombus dislodgement or escape from the second mesh openings 131b, thus further reducing the risk of thrombus dislodgement or escape.

The first thrombectomy stent 131 includes a proximal portion 1311, a middle portion 1312 and a distal portion 1313, which are joined sequentially. A number of first mesh openings 131a are arranged on the proximal and distal portions 1311, 1313. These first mesh openings 131a may be distributed in the first thrombectomy stent 131, uniformly or not, preferably uniformly. The first mesh openings 131a are arranged along the axis and circumference of the first thrombectomy stent 131 . The first mesh openings 131a can provide certain radial support, allowing their struts 13A to be more easily embedded into a soft thrombus. Further, the first mesh openings 131a in the distal portion 1313 of the first thrombectomy stent 131 can provide certain thrombus capture capabilities. The first mesh openings 131a in the distal portion 1313 may be equal to area (i.e., size) to those in the proximal portion 1311, or not. Preferably, the first mesh openings 131a in the distal portion 1313 have a smaller area than the first mesh openings 131a in the proximal portion 1311. This enables the first mesh openings 131a in the distal portion 1313 to better capture a thrombus contained in the first thrombectomy stent 131. Optionally, the first mesh openings 131a may have an area of 0.7 mm² to 4.0 mm².

The second mesh openings 131b have an area much larger than an area of the first mesh openings 131a. Here, by "much larger than", it is intended to mean that the area of the second mesh openings 131b is at least 2.5 times the area of the first mesh openings 131a. The present application is not limited to any particular area ratio of the second mesh openings 131b to the first mesh openings 131a, and those skilled in the art can appropriately determine it, as practically need. Preferably, the area of the second mesh openings 131b is 2.5 to 6 times the area of the first mesh openings 131a. Further, a number of second mesh openings 131b are distributed in the middle portion 1312 right at locations wherein the thrombi accumulate.

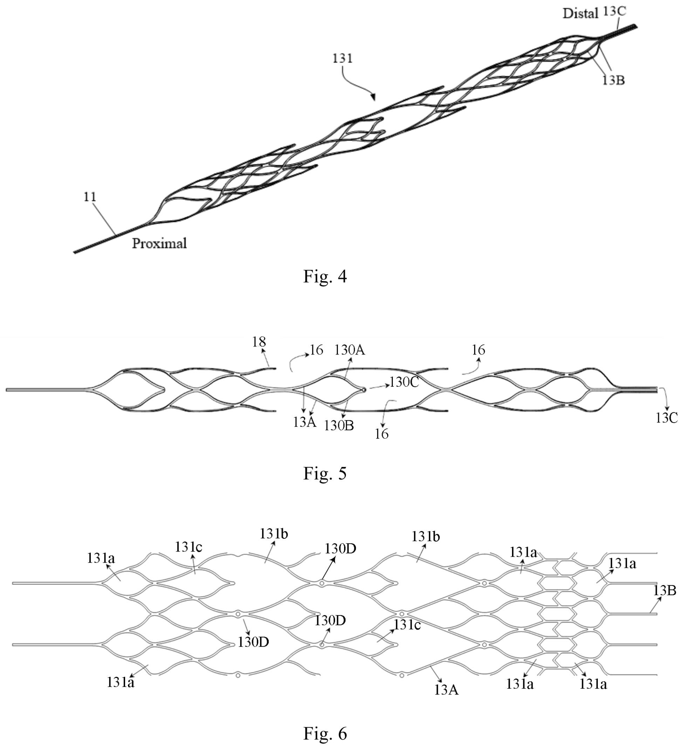

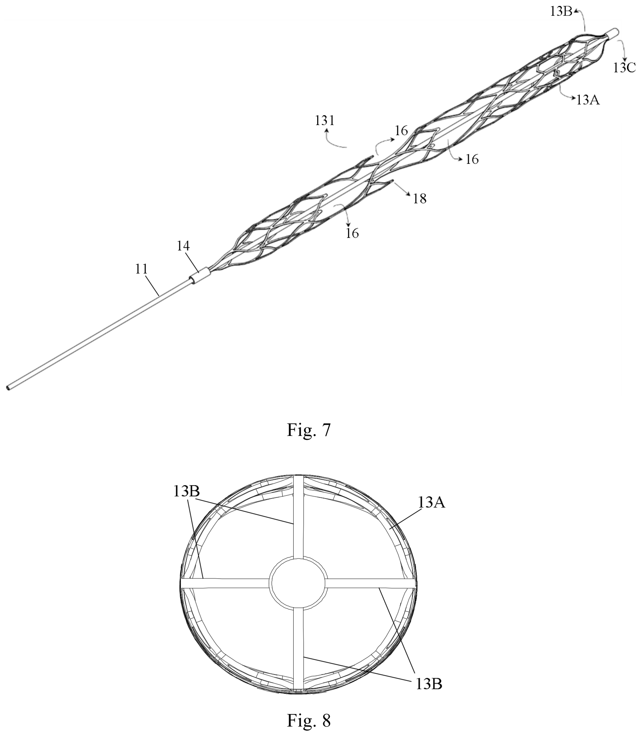

Referring to Figs. 5 and 7, multiple second mesh openings 131b with large-area opening inlet 16 are formed in the exterior surface of the first thrombectomy stent 131. The opening inlets 16 may be so large that most hard and large soft thrombi can embed into the second mesh openings, or even that such thrombi can entirely pass through the opening inlets 16 into the interior of the first thrombectomy stent 131. Thus, a thrombus will be less likely to come into contact with a wall of a blood vessel. Moreover, during retrieval, large intact soft thrombi may be retained at opening inlets 16, and small disrupted soft thrombus and hard thrombi can entirely pass through second mesh openings 131b into the interior of the first thrombectomy stent 131 and move therein to the distal end of the first thrombectomy stent 131. Due to a higher density of the first mesh openings 131a in the distal portion 1313 than a density of the opening inlets 16 (i.e., of the second mesh openings 131b), the thrombi or thrombus will come into contact with the first thrombectomy stent 131 over larger areas and with increased friction at the distal end. Thus, the thrombus adheres to the inner wall at the distal end of the first thrombectomy stent 131, thereby achieving an interception effect.

It should be noted that there are no fewer than, preferably two to six opening inlets 16, at least two opening inlets 16 are arranged at an interval on the exterior surface of the first thrombectomy stent 131. Each of the opening inlets 16 is formed by one second mesh opening 131b and in communication with the interior of the first thrombectomy stent 131.

In further embodiments, a plurality of second mesh openings 131b may be distributed on different circumferences of the first thrombectomy stent 131. These second mesh openings 131b on different circumferences may have overlap portions along the axis and/or circumferential of the first thrombectomy stent 131. In this way, a blind zone for thrombectomy can be reduced, increasing the probability of successfully capturing large and hard thrombi through releasing the thrombectomy stent 13 only once. This can result in increased thrombectomy efficiency, shorten the time required for completing an associated surgical procedure, and reduce patient trauma. Specifically, when viewed from one end of the thrombectomy device 10 to its other end, the plurality of second mesh openings 131b may have overlap portions. That is, if any of the second mesh openings 131b were translated along the axis of the thrombectomy device 10 toward another second mesh opening 131b, it would surely cross the other second mesh opening 131b at a circumferential location of the thrombectomy device 10, i.e., it would surely overlap part of the other second mesh opening 131b at the circumferential location. If any of the second mesh openings 131b were translated along the axis of the thrombectomy device 10 toward another second mesh opening 131b, it would cross the other second mesh opening 131b at a circumferential location of the thrombectomy device 10, and the cross part is the overlap portion.

For ease of understanding, reference is made to Fig. 3, which shows a flattened view of the first thrombectomy stent 131. In the illustrated example, there are two second mesh openings 131b on each circumference. When viewed from one end of the first thrombectomy stent 131 to its other end, there are overlap portions 17 of the second mesh openings 131b between distal and proximal circumferences (as indicated by the regions bounded by the dashed lines). Moreover, each second mesh opening 131b in each circumference has overlap portions 17 respectively with the two adjacent second mesh openings 131b in another circumference. Thus, second mesh openings 131b on different circumferences have overlap portions along the axis and circumference of the first thrombectomy stent 131.

Preferably, the opening inlets 16 are distributed uniformly both along the axis and circumference of the first thrombectomy stent 131 and span the entire circumference of an outer wall of the first thrombectomy stent 131. The opening inlets 16 are continuously arranged along the corresponding thrombectomy length range. With this arrangement, a blind zone for thrombectomy can be significantly reduced, and in a thrombectomy procedure, once any large thrombus comes into contact with the outer wall of the first thrombectomy stent 131 that has fully expanded, no matter where the contact occurs, it will always be trapped into the interior of the first thrombectomy stent 131 by an opening inlet 16 in the outer wall of the first thrombectomy stent 131. This can reduce the translational and rotational movements, achieving higher thrombectomy efficiency. Each second mesh opening 131b may have a circumferential length L, which is approximately half of the perimeter of the first thrombectomy stent 131. Accordingly, at least two second mesh openings 131b can span an entire circumference of the exterior surface of the first thrombectomy stent 131.

Further, the third mesh opening 131c may be formed in the following way. A free projection 18 extends from a stent 13A at one side into the second mesh opening 131b is provided in the second mesh opening 131b. The free projection comprises a closed mesh opening structure and forms the third mesh opening 131c. The free projection 18 may be formed either by extending a strut 13A at the proximal end of a second mesh opening 131b distally into the second mesh opening 131b, or by extending a strut 13A at its distal end proximally into the second mesh opening 131b. With this arrangement, during expansion after the thrombectomy device 10 is released, a thrombus can enter the interior of the first thrombectomy stent 131 through an outside area of a third mesh opening 131c in a second mesh opening 131b. Moreover, during retraction of the thrombectomy device 10, the free projections 18 of the third mesh opening 131c can block the escape of the thrombus from the second mesh opening 131b. This facilitates thrombus retention in, and reduces the risk of thrombus dislodgement from, the thrombectomy device 10 during its advancement in a tortuous blood vessel.

The third mesh openings 131c delimited by the free projections 18 have a much smaller area than the second mesh openings 131b. It will be understood that the third mesh openings 131c may have their own struts 13A independent of the second mesh openings 131b. Each third mesh opening 131c may have a first strut 130A and a second strut 130B, each connected at one end to a strut 13A of the second mesh opening 131b and at the other end to the other one of them to form the aforementioned free projections 18 that extends distally or proximally, as particularly shown in Fig. 5.

Each third mesh opening 131c may have such a length that does not interfere with the entry of a thrombus (in particular, a large thrombus) into the interior of the first thrombectomy stent 131. Optionally, an axial length of the third mesh openings 131c may be 1/4 to 1/2 of an axial length of the second mesh openings 131b.

Further, in order to accurately locate the second mesh openings 131b, a position of the first thrombectomy stent 131 corresponding to the second mesh openings 131b may be provided with radiopaque elements (not labeled. These radiopaque elements may be impenetrable to X-rays, enabling one to clearly determine whether and how well the first thrombectomy stent 131 contacts a thrombus in a blood vessel. The thrombectomy device 10 may be displaced or rotated to accurately align a second mesh opening 131b with a large thrombus, resulting in improved thrombectomy efficiency.

In one embodiment, as shown in Figs. 3 and 5, the radiopaque elements may be arranged in recesses formed at the joints 130C of the struts 130A and 130B in third mesh openings 131c. In an alternative embodiment, as shown in Fig. 6, the radiopaque elements may be arranged in recesses formed at the joints 130D of two struts 13A of adjacent second mesh openings 131b. The present application is not limited to any particular shape of such recesses, and circular recesses are preferred because they can be easily formed. In still alternative embodiments, the radiopaque elements may be provided as a radiopaque wire wound on struts 13A of second mesh openings 131b. For example, one or more radiopaque wires may be helically wound on struts 13A of second mesh openings 131b. The present application is not limited to any particular material of the radiopaque elements, and possible examples include, but are not limited to, platinum, gold, platinum-iridium alloys, tantalum and other radiopaque metals.

With this arrangement, as a result of retracting the microcatheter 100, the thrombectomy device 10 may gradually self-expand into contact with a thrombus 101. At this point, an imaging device may be used to observe the positions of the radiopaque elements of the second mesh openings 131b. As a result, it can be determined whether the large thrombus 101 has completely entered the interior of the first thrombectomy stent 131. If required, a translation along the axis of the thrombectomy device 10, or a rotation by a small angle, may be made to ensure that the thrombus 101 enters the interior through an opening inlet 16.

Further, the second thrombectomy stent 132 may be formed by braiding a metal or polymer wire with hyperelasticity and good shape memory properties, which has been set in shape by undergoing a thermal process in a mold, then a braided mesh with a desired shape and dimensions can be obtained. Preferably, the second thrombectomy stent 132 is a formed by braiding wires of a shape memory alloy and a radiopaque precious metal, which can help a surgeon determine the position of the second thrombectomy stent 132 in a blood vessel, thereby resulting in improved surgical efficiency and accuracy.

The second thrombectomy stent 132 may be in the form of a disc-shaped mesh, but the present application is not limited to any particular shape. A shape allowing minimized contact with an inner wall of a blood vessel is selected, such as spherical, disc- or basket-like. When in an expanded configuration during use, the second thrombectomy stent 132 is preferred to come into contact, preferably line contact with an inner wall of a blood vessel over a small contact area, resulting in minimal damage to the blood vessel. Compared with the first thrombectomy stent 131, the second thrombectomy stent 132 contains denser closed mesh openings and therefore can capture thrombus fragments resulting from thrombectomy of the first thrombectomy stent 131.

Preferably, the second thrombectomy stent 132 consists of a braid wire with a diameter smaller than the width of the struts 13A in the first thrombectomy stent 131. Since the second thrombectomy stent 132 functions mainly for thrombus capture and is therefore not desired to provide much radial support, relatively thin braid wire may be selected to form braided dense mesh. However, if the second thrombectomy stent 132 contains too dense mesh openings, it may be difficult to crimp into the microcatheter 100, and thrombus fragments attached to the second thrombectomy stent 132 may be easily scraped off by the catheter. In view of this, the second thrombectomy stent 132 preferably consists of a braid wire with a diameter of 0.02 mm to 0.12 mm and contains mesh openings with an area of 0.05 mm² to 1 mm².

In summary, the proposed thrombus removal system and assisted retrieval system offer at least the following advantages over the prior art:
(1) The self-expanding stent in the assisted retrieval system can cooperate with the thrombectomy device by allowing the thrombectomy device to be at least partially retracted into the self-expanding stent after it captures a thrombus. In this way, the thrombectomy device can be retracted from an occluded site into the retrieval catheter while being protected by the self-expanding stent. As such, a thrombus that falls off or dislodges from the thrombectomy stent during the retraction can be effectively prevented from entering a branch or distal blood vessel, resulting in higher thrombectomy efficiency and success, a reduced risk of secondary stroke and increased surgical safety.
   Through retracting the thrombectomy device under the protection of the self-expanding stent, a thrombus that falls off during the retraction can be desirably prevented from entering a branch or distal vessel.
(2) The self-expanding stent may comprise a slit extending along its axis, which allows the self-expanding stent to adapt its shape and size to tubular lumens with different diameters, such as differently sized retrieval catheters and blood vessels. This enables the self-expanding stent to be used in a wider range of applications, adding flexibility and convenience to the use of the assisted retrieval system. In particular, when placed into a tubular lumen, the self-expanding stent may be adaptively rolled to be partially overlapped, or unrolled so as to no longer have any overlap portions. With this arrangement, first, the self-expanding stent can be received in a retrieval catheter with a smaller diameter. Therefore, it can be applied to a blood vessel with a smaller diameter, and advanced to a deeper location in a blood vessel. Second, when used in a blood vessel with a smaller tubular lumen, the self-expanding stent may be rolled and overlapped. On the contrary, when used in a blood vessel with a larger tubular lumen, it may comprise an "unrolled" configuration. These make it suitably usable in blood vessels with various different diameters. Third, radial support of the self-expanding stent can be weakened, making it cause less damage to an inner wall of a blood vessel during retraction therein.
(3) In the thrombectomy device, the first thrombectomy stent consists of multiple types of mesh openings, which allow the first thrombectomy stent to effectively remove soft and hard thrombi with higher thrombectomy efficiency and success. In particular, the first mesh opening comprises a relative small area, which can easily embed into a soft thrombus. The second mesh opening 131b comprises a large-area opening, which allow the hard and soft thrombi to embed into the second mesh openings or to be trapped into the interior of the first thrombectomy stent. At the same time, the third mesh openings form a spatially wrap structure in the second mesh openings to mitigate thrombus dislodgement or escape from the second mesh openings.
(4) The second mesh openings in the first thrombectomy stent may have overlap portions both along the axis and circumference of the first thrombectomy stent. This arrangement results in a reduced blind zone, increasing the probability of successfully capturing large and hard thrombi through releasing the thrombectomy stent only once. This can result in increased thrombectomy efficiency, shorten the time required for completing an associated surgical procedure, and reduce patient trauma.
(5) A position of the first thrombectomy stent corresponding to the second mesh openings may be provided with radiopaque markers. These radiopaque markers may be impenetrable to X-rays and thereby enable accurate locating of the second mesh openings, ensuring that a target large thrombus can embed into the first thrombectomy stent or be trapped into the interior of the first thrombectomy stent.
(6) The thrombectomy device may further include a second thrombectomy stent in the form of a braided dense mesh, which can be used in combination with the first thrombectomy stent to achieve effective capture of an escaping thrombus or thrombus fragments.

Finally, it should be noted that, as discussed herein, a "target object" may be any form of occluding matter, such as a red soft thrombus, a white hard thrombus, or another type of clot. In addition, a "patient" or "subject" may be a human or any animal. It should be appreciated that an animal may be a variety of any applicable type, including, but not limited thereto, mammal, veterinarian animal, livestock animal or pet type animal, etc. As an example, the animal may be a laboratory animal specifically selected to have certain characteristics similar to human (e.g. rat, dog, pig, monkey), etc.

The description presented above is merely that of some preferred embodiments of the present invention and is not intended to limit the scope thereof in any sense. Any and all changes and modifications made by those of ordinary skill in the art based on the above teachings fall within the scope of the invention.

## Claims

1. A thrombus removal system, comprising:
a thrombectomy device comprising a push-pull guide wire and a thrombectomy stent that are sequentially coupled along an axis of the thrombectomy device; and
an assisted retrieval system comprising a retrieval catheter, a push-pull mechanism and a self-expanding stent, wherein a proximal end of the self-expanding stent is coupled to a distal end of the push-pull mechanism, wherein the push-pull mechanism is configured to be slidably disposed within the retrieval catheter and to push or pull the self-expanding stent along an axis of the retrieval catheter, wherein the self-expanding stent comprises a collapsed configuration during delivery thereof in the retrieval catheter and an expanded configuration when being partially released from the retrieval catheter,
wherein the assisted retrieval system is configured, after the thrombectomy stent captures a target object, to be moved along the push-pull guide wire and to be located on a proximal side of the thrombectomy stent, thereby enabling the self-expanding stent to be partially released from the retrieval catheter to expand,
wherein the thrombectomy stent is configured, after capturing the target object, to be pulled by the push-pull guide wire proximally toward the assisted retrieval system, and to be at least partially retracted into the self-expanding stent through a distal opening of the self-expanding stent that has been expanded for retrieval.

2. The thrombus removal system according to claim 1, wherein after the thrombectomy stent is at least partially retracted into the self-expanding stent, the thrombectomy device and the assisted retrieval system are configured to be proximally moved together into an outer sheath, or wherein the thrombectomy stent is proximally moved and passed through the self-expanding stent into the retrieval catheter.

3. The thrombus removal system according to claim 1 or 2, wherein the self-expanding stent consists of a braided mesh and comprises a slit extending through the self-expanding stent along an axis thereof.

4. The thrombus removal system according to claim 3, wherein the braided mesh is switchable between a rolled configuration and an unrolled configuration, making the self-expanding stent able to adapt a shape based on a size of a tubular lumen where the self-expanding stent is located.

5. The thrombus removal system according to claim 4, wherein the self-expanding stent comprises a crimped section, a tapered section and a cylindrical support section, that are sequentially joined along the axis of the self-expanding stent, wherein the crimped section is disposed in the retrieval catheter and is coupled to the push-pull mechanism, and wherein the tapered and support sections can be released from the retrieval catheter to expand.

6. The thrombus removal system according to claim 5, wherein a plurality of radiopaque markers arranged circumferentially are provide at an edge of a proximal end of the tapered section.

7. The thrombus removal system according to claim 1 or 2, wherein the push-pull mechanism comprises a push rod coupled at a distal end thereof to a proximal end of the self-expanding stent, or wherein the push-pull mechanism comprises a push rod and a pull wire, wherein a distal end of the pull wire is coupled to the proximal end of the self-expanding stent, and wherein a proximal end of the pull wire passes through the push rod and extends to a proximal end of the retrieval catheter.

8. The thrombus removal system according to claim 1 or 2, wherein at least a part of an exterior surface of the self-expanding stent is provided with a hydrophilic coating layer, and/or wherein an inner wall of a distal end of the retrieval catheter is provided with a hydrophilic coating layer.

9. The thrombus removal system according to claim 1 or 2, wherein the thrombectomy stent comprises a first thrombectomy stent, wherein a proximal end of the first thrombectomy stent is coupled to a distal end of the push-pull guide wire, wherein the first thrombectomy stent comprises a tubular structure consisting of a plurality of interconnected closed mesh openings, wherein the closed mesh openings include first mesh openings, second mesh openings and third mesh openings, wherein the first and third mesh openings have a smaller area than the second mesh opening, wherein the first mesh openings has no overlap with respect to the second or third mesh openings, wherein the third mesh opening is arranged within the second mesh opening, and wherein at least two second mesh openings span at least a circumference of an exterior surface of the first thrombectomy stent.

10. The thrombus removal system according to claim 9, wherein the second mesh opening has an area which is 2.5 to 6 times an area of the first mesh opening.

11. The thrombus removal system according to claim 9, wherein a position of the first thrombectomy stent corresponding to the second mesh opening is provided with a radiopaque element.

12. The thrombus removal system according to claim 9, wherein the first thrombectomy stent comprises a proximal portion, a middle portion and a distal portion, which are joined sequentially, wherein the first mesh openings are distributed on the proximal portion and the distal portion, wherein the second mesh openings are distributed on the middle portion, and wherein the first mesh opening in the distal portion is smaller in area than the first mesh opening in the proximal portion.

13. The thrombus removal system according to claim 9, wherein a plurality of second mesh openings are distributed on different circumferences of the first thrombectomy stent, and wherein the second mesh openings distributed on different circumferences have overlap portions along an axis and/or circumference of the first thrombectomy stent.

14. The thrombus removal system according to claim 9, wherein the thrombectomy stent further comprises a second thrombectomy stent formed by a braided dense mesh, wherein a proximal end of the second thrombectomy stent is flexibly coupled to a distal end of the first thrombectomy stent, wherein the second thrombectomy stent is able to occlude the self-expanding stent on a distal side of the first thrombectomy stent after the first thrombectomy stent being retracted into the self-expanding stent.

15. The thrombus removal system according to claim 14, wherein the second thrombectomy stent is configured to occlude a distal opening of the self-expanding stent at a distal side of the self-expanding stent, or wherein the second thrombectomy stent is at least partially retracted into the self-expanding stent for occlusion.

16. An assisted retrieval system, comprising a retrieval catheter, a push-pull mechanism and a self-expanding stent, wherein a proximal end of the self-expanding stent is coupled to a distal end of the push-pull mechanism, wherein the push-pull mechanism is configured to be slidably disposed within the retrieval catheter and to push or pull the self-expanding stent along an axis of the retrieval catheter, wherein the self-expanding stent comprises a collapsed configuration during delivery thereof in the retrieval catheter and an expanded configuration when being partially released from the retrieval catheter,
wherein the assisted retrieval system is configured to, after a thrombectomy stent captures a target object, to be moved along a push-pull guide wire and to be located on a proximal side of the thrombectomy stent, thereby enabling the self-expanding stent to be partially released from the retrieval catheter to expand and enabling at least a part of the self-expanding stent to be retracted into the self-expanding stent through a distal opening of the self-expanding stent that has been expanded for retrieval.

17. The assisted retrieval system according to claim 16, wherein the self-expanding stent consists of a braided mesh and comprises a slit extending through the self-expanding stent along an axis thereof.

18. The assisted retrieval system according to claim 17, wherein the braided mesh is switchable between a rolled configuration and an unrolled configuration, making the self-expanding stent able to adapt a shape based on a size of a tubular lumen where the self-expanding stent is located.
